# DEMANDE DE BREVET EUROPEEN

(11) **EP 2 529 703 A1**
(43) Date de publication de la demande: **05.12.2012**
(21) Numéro de dépôt: 11305682.4
(22) Date de dépôt: 03.06.2011
(51) Int. Cl.: A61F 2/36

(54) **Prothese de hanche**

(71) Demandeur: Bourse, Philipp, 31100 Toulouse (FR)
(72) Inventeur: Bourse, Philipp, 31100 Toulouse (FR)
(74) Mandataire: Blot, Philippe Robert Emile

(57) **Abrégé**

L'invention concerne une prothèse de hanche (12) comprenant :
- une partie d'ancrage (14) adaptée pour être ancrée dans un col de fémur (6) et comprenant un tube (18) comprenant un premier tronçon 25 taraudé adapté pour s'étendre dans le col de fémur (6) le long d'un axe (A-A),
- une tête sphérique (17) destinée à remplacer la tête de fémur (4), et
- une partie articulée (16) comprenant une tige filetée (46) de réglage s'étendant le long de l'axe (A-A) adaptée pour être vissée et/ou dévissée dans le tube (18), pour régler la position du centre de rotation de la tête sphérique (17) par rapport à la partie d'ancrage (14),

caractérisée en ce que la partie d'ancrage (14) est prévue pour s'étendre uniquement dans le col anatomique du fémur (6).

## Description

La présente invention concerne une prothèse de hanche du type comprenant une partie d'ancrage adaptée pour être ancrée dans un col de fémur et comprenant un tube comprenant un premier tronçon taraudé adapté pour s'étendre dans le col de fémur le long d'un axe, une tête sphérique destinée à remplacer la tête de fémur, et une partie articulée comprenant une tige filetée de réglage s'étendant le long de l'axe adaptée pour être vissée et/ou dévissée dans le tube, pour régler la position du centre de rotation de la tête sphérique par rapport à la partie d'ancrage.

Des prothèses de hanche sont utilisées pour le traitement de l'arthrose.

FR 2 438 470 divulgue une prothèse de hanche du type précité pour une fracture cervicale et dans laquelle la partie d'ancrage est volumineuse et nécessite une intervention chirurgicale compliquée sollicitant les ligaments et les muscles.

Par conséquent, le patient recevant une telle prothèse de hanche nécessite de soins adaptés après l'intervention chirurgicale qui sont longs et surtout très coûteux.

La présente invention a pour but de remédier à ces inconvénients.

A cet effet, l'invention a pour objet une prothèse du type précité et caractérisée en ce que la partie d'ancrage est prévue pour s'étendre uniquement dans le col anatomique du fémur.

Selon d'autres modes de réalisation, l'invention comporte l'une ou plusieurs des caractéristiques suivantes :
- la tige filetée est prévue pour s'étendre uniquement dans le col anatomique ;
- la partie articulée comprend une tige de connexion adaptée pour recevoir la tête sphérique, et la tige filetée et la tige de connexion sont rectilignes et d'un seul tenant ;
- le tube comprend un deuxième tronçon lisse et la partie articulée comprend une tige de connexion, la tige de connexion étant adaptée pour être insérée et pour coulisser dans le deuxième tronçon suivant l'axe et étant en appui axial contre la tige filetée ;
- la tige de connexion est indexée angulairement de telle sorte que la tige de connexion est adaptée pour se déplacer linéairement le long de l'axe sans tourner autour de l'axe lorsque la tige filetée est vissée ou dévissée dans le tube ;
- la tige filetée et la tige de connexion s'étendent suivant un même axe ;
- la tige de connexion présente une forme coudée ;
- la tige filetée comporte à une seconde extrémité une empreinte adaptée pour coopérer avec un outil pour visser et dévisser la tige filetée dans le tube et pour coopérer avec un canon d'une prothèse complémentaire du type vis plaque, en cas d'une fracture pertrochantérienne après la pose de la prothèse ;
- la prothèse comprend un élément de blocage adapté pour bloquer la position de la tige filetée par rapport au tube, notamment après réglage de la position du centre de rotation de la tête sphérique ;
- la partie d'ancrage comprend une plaque d'appui adaptée pour prendre appui sur une surface de résection du col du fémur résultant de la résection de la tête de fémur ;
- le tube comporte sur sa surface extérieure une pièce de stabilisation primaire adaptée pour bloquer une rotation du tube autour de l'axe par rapport au col de fémur et/ou un pivotement du tube par rapport au col de fémur ; et
- la partie d'ancrage est au moins en partie recouverte d'un revêtement ostéoinducteur.

L'invention a en outre pour objet une méthode de pose d'une prothèse de hanche comprenant les étapes successives suivantes :
- installer un patient en décubitus dorsal ;
- introduire une broche guide de 1,5 à 2,5 mm de diamètre sous contrôle radioscopique dans un fémur selon l'axe cervical d'une face externe du fémur sous le massif trochantérien à travers le massif trochantérien centralement dans le col anatomique jusqu'au centre de rotation de la tête de fémur ;
- mesurer des distances qui serviront au réglage d'une longueur de la prothèse de hanche :
   a) de la corticale du point de pénétration de la broche guide au centre de rotation de la tête de fémur ;
   b) de la ligne intertrochantérienne à une zone de coupe du col, ultérieurement présentant une surface de résection ;
   c) de la zone de coupe du col au centre de rotation de la tête de fémur 4 ;
- enfiler une tarrière cylindrique motorisée sur la broche guide qui, guidée par la broche guide, fraise un canal dans le fémur ;
- sectionner, par voie antérieure, la tête de fémur par une scie oscillante et former la surface de résection sur laquelle prendra appui ultérieurement une plaque d'appui, caractérisée en ce que la prothèse de hanche est une prothèse de hanche selon l'invention.

Selon d'autres modes de réalisation, la méthode comporte l'une ou plusieurs des étapes suivantes :
- introduire une partie d'ancrage par voie antérieure dans le canal et ancrer la partie d'ancrage dans le col de fémur, de sorte que la plaque d'appui prend appui sur la surface de résection ;
- introduire au moins partiellement une partie cylindrique d'une tige de connexion d'une partie articulée dans la partie d'ancrage par voie antérieure et visser une tige filetée de la partie articulée dans un tube taraudé de la partie d'ancrage dans un sens opposé afin d'approcher la tige filetée à la tige de connexion ;
- glisser une tête sphérique par voie antérieure et positionner la tête sphérique sur un cône morse de la tige de connexion ;
- ajuster par vissage et/ ou dévissage de la tige filetée, la position de la partie articulée par rapport à la partie d'ancrage qui détermine la position du centre de rotation de la tête sphérique et la longueur de la prothèse ;
- remesurer les distances a), b) et c) ;
- enfoncer en étant guidée par la broche guide une pièce de stabilisation primaire autour du tube taraudé ;
- bloquer la partie articulée par rapport à la partie d'ancrage au moyen d'un élément de blocage une fois que la longueur de la prothèse est ajustée.

L'invention sera mieux comprise à la lecture de la description qui va suivre, donnée uniquement à titre d'exemple et faite en se référant aux dessins annexés sur lesquels :
- la figure 1 est une vue schématique antérieure d'une partie supérieure du fémur comprenant la tête de fémur et le col du fémur,
- la figure 2 est une vue analogue à celle de la figure 1 dans laquelle est posée une prothèse de hanche selon l'invention qui remplace la tête du fémur,
- la figure 3 est une coupe transversale d'une partie d'ancrage et d'une partie articulée de la prothèse de hanche selon l'invention,
- la figure 4 est une vue le long de l'axe A-A de la partie d'ancrage de la figure 3 et
- les figures 5 et 6 sont des vues analogues à celle de la figure 3 d'une prothèse de hanche selon deux variantes.

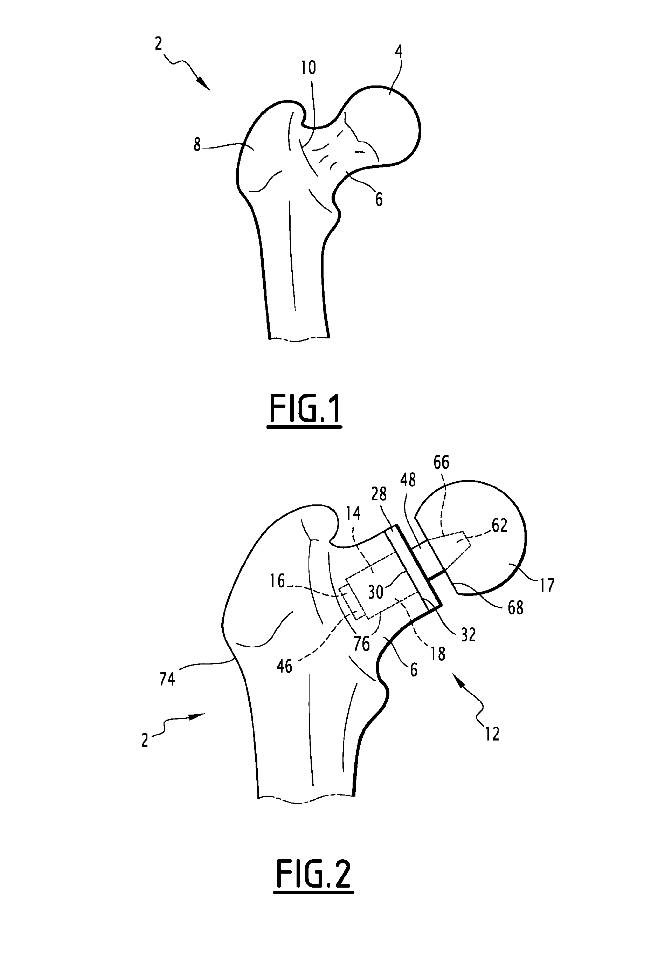

Sur la figure 1 est représentée une vue antérieure d'une partie supérieure d'un fémur 2 intact comprenant la tête de fémur 4, le col de fémur 6 et le massif trochantérien 8.

Le col de fémur 6 relie la tête de fémur 4 et le massif trochantérien 8.

Le col de fémur 6 désigne ici le col de fémur dit « anatomique » qui s'étend exclusivement entre la tête de fémur 4 et le massif trochantérien 8 dont la partie supérieure est représentée par la ligne inter-trochantérienne 10, par opposition au col de fémur dit « chirurgical » qui comprend le col du fémur 6 anatomique et le massif trochantérien 8.

La tête de fémur 4 forme avec un cotyle (non représenté) une articulation.

Sur la figure 2 est représentée une prothèse de hanche 12 selon l'invention posée dans le col de fémur 6 et remplaçant la tête de fémur 4.

La prothèse de hanche 12 s'étend uniquement dans le col de fémur 6 anatomique.

La prothèse de hanche 12 comporte une partie d'ancrage 14 adaptée pour être ancrée dans le col de fémur 6, une partie articulée 16 adaptée pour être mobile par rapport à la partie d'ancrage 14 et une tête sphérique 17 adaptée pour remplacer la tête de fémur 4.

La partie d'ancrage 14 s'étend le long d'un axe longitudinal A-A. Elle est adaptée pour s'étendre uniquement dans le col de fémur 6 anatomique. Elle présente une longueur axiale limitée. La partie d'ancrage 14 est métallique.

La partie d'ancrage 14 comporte un tube 18 adapté pour s'étendre dans le col de fémur 6. Le tube 18 s'étend le long de l'axe A-A. Le tube 18 comporte un alésage 20 traversant le long de l'axe A-A, une surface intérieure 22 et une surface extérieure 24.

L'alésage 20 comprend un premier tronçon 25 taraudé muni d'un taraudage 26 et un deuxième tronçon 27 lisse.

La surface extérieure 24 est recouverte d'un revêtement ostéoinducteur adapté pour promouvoir la croissance du tissu osseux sur la surface extérieure 24 du tube 18, une fois que la prothèse de hanche 12 est posée dans le col de fémur 6 anatomique.

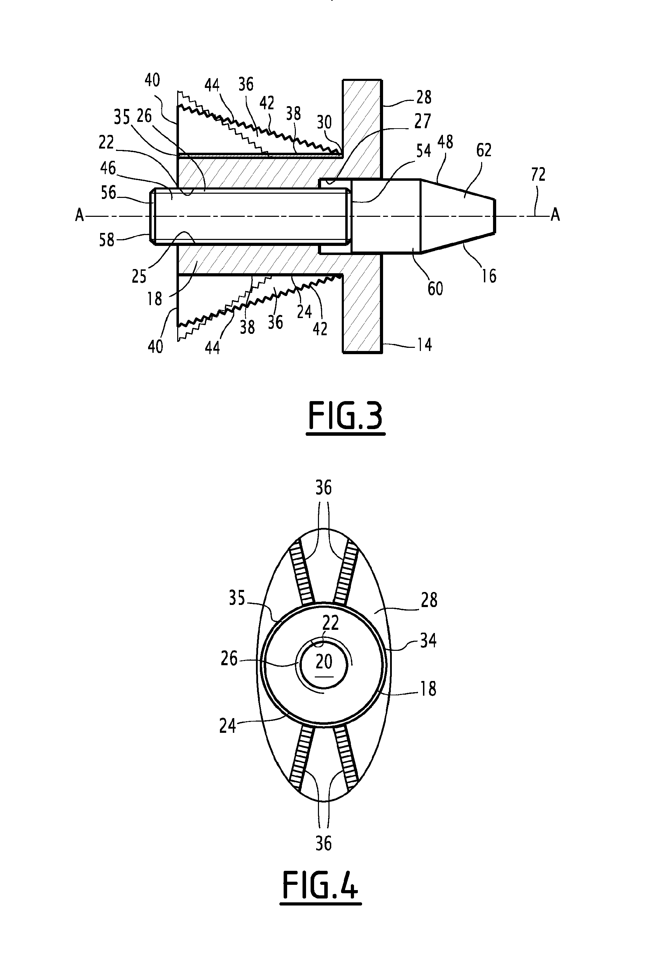

La partie d'ancrage 14 comporte également une plaque d'appui 28. La plaque d'appui 28 s'étend dans un plan sensiblement perpendiculaire à l'axe A-A. Dans l'exemple représenté sur la figure 4, la plaque d'appui 28 présente une forme d'ellipse. En variante, la forme de la plaque d'appui 28 peut être différente, par exemple ronde. La plaque d'appui 28 est disposée à une première extrémité 30 du tube 18. La première extrémité 30 est celle qui, une fois que la partie d'ancrage 14 est ancrée dans le col de fémur 6, est adjacente à une surface de résection 32 du col de fémur 6 résultant de la résection de la tête de fémur 4. La plaque d'appui 28 prend appui sur ladite surface de résection 32 du col de fémur 6. La surface de la plaque d'appui 28 orientée vers la surface de résection 32 du col de fémur 6 est également recouverte du revêtement ostéoinducteur. Le tube 18 et la plaque d'appui 28 sont venus de matière.

La partie d'ancrage 14 comporte en outre une pièce de stabilisation primaire 34. La pièce de stabilisation primaire 34 est recouverte du revêtement ostéoinducteur. La pièce de stabilisation primaire 34 comporte un manchon 35 et au moins une ailette 36. Le manchon 35 est adapté pour recevoir le tube 18 de sorte à l'immobiliser l'un par rapport à l'autre par friction. Les ailettes 36 sont disposées en surface extérieure du manchon 35 orientée à l'opposé du tube 18 et sensiblement dans des mêmes plans que l'axe A-A.

Dans l'exemple représenté sur la figure 4, la pièce de stabilisation primaire 34 comporte quatre ailettes 36. Néanmoins, le nombre des ailettes 36 n'est pas limité à quatre mais peut adopter d'autres valeurs appropriées. Les ailettes 36 se présentent par exemple sous forme sensiblement triangle rectangle.

L'ailette 36 en forme triangle rectangle comporte deux cathètes 38, 40 et une hypothénuse 42. Dans une première orientation (figure 3) pour chaque ailette 36, la cathète 38 est adjacente au tube 18 et la cathète 40 est libre orientée à l'opposé de la première extrémité du tube 18.

L'hypoténuse 42 et la cathète 40 comportent des dents 44. Les dents 44 sont adaptées pour augmenter la surface du bord libre de la pièce 34 de stabilisation primaire. En variante, l'hypothénuse 42 et la cathète 40 sont lisses.

La première orientation des ailettes 36 est adaptée pour bloquer la rotation du tube 18 autour de l'axe A-A par rapport au col de fémur 6 et le pivotement du tube 18 par rapport au col de fémur 6.

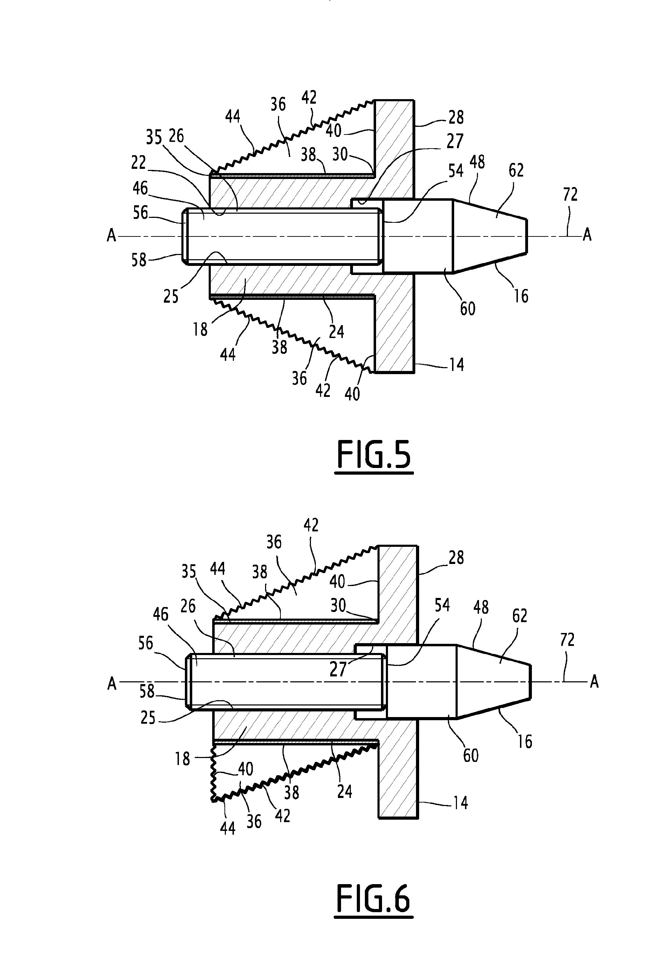

Dans une deuxième orientation (figure 5) pour chaque ailette 36, les deux cathètes 38, 40 sont adjacentes au tube 18 et à la plaque d'appui 28, respectivement.

La deuxième orientation des ailettes 36 est adaptée pour bloquer la rotation du tube 18 autour de l'axe A-A par rapport au col de fémur 6.

Dans une troisième orientation, les ailettes 34 sont orientées alternativement entre la première orientation et la deuxième orientation (figure 6). C'est-à-dire la cathète 38 est toujours adjacente au tube 18, alors que la cathète 40 alterne entre une position adjacente à la plaque d'appui 28 et une position libre orientée à l'opposé de la première extrémité 30 du tube 18.

La troisième orientation des ailettes 36 est adaptée pour bloquer la rotation du tube 18 autour de l'axe A-A par rapport au col de fémur 6 et le pivotement du tube 18 par rapport au col de fémur 6.

Comme représente sur la figure 3, les longueurs des cathètes 38, 40 sont variables. Dans le cas d'un col de fémur 6 dit « évasé », la cathète 40 est longue, souvent aussi longue que le rayon libre de la plaque d'appui 28 (représenté en lignes fines sur la figure 3). Dans ce cas, la cathète 38 est souvent plus courte que le tube 18. L'angle formé entre la cathète 38 et l'hypoténuse 42 est compris entre 20 et 60°.

Dans le cas d'un col de fémur 6 dit « cylindrique », la cathète 40 est courte, plus courte que le rayon libre de la plaque d'appui 28 (représenté en lignes épais sur la figure 3). Dans ce cas, la cathète 38 est souvent longue, quasiment aussi longue que le tube 18. L'angle formé entre la cathète 38 et l'hypoténuse 42 est compris entre 5 et 20°.

Parmi les trois orientations précitées, la première orientation d'ailettes 36 est l'orientation préférée.

La partie d'ancrage 14 est adaptée pour être ancrée dans le col de fémur 6 sans utilisation de ciment, par exemple par impact contre la partie d'ancrage 14. Dans certains cas, par exemple si la prothèse de hanche 12 est utilisée pendant une durée courte d'ordre de cinq ans ou moins, la partie d'ancrage 14 peut être néanmoins cimentée.

La partie articulée 16 comporte une tige filetée 46 et une tige de connexion 48. La partie articulée 16 est mobile en translation le long de l'axe A-A par rapport à la partie d'ancrage 14. La partie articulée 16 comporte une perforation centrale, coaxiale à l'axe A-A et présentant un diamètre entre 2 et 3 mm. La perforation centrale est adaptée pour recevoir une broche guide 72 (voir plus bas).

La tige filetée 46 est adaptée pour s'étendre uniquement dans le col de fémur 6 anatomique.

La tige filetée 46 est cylindrique et comporte un filetage 52, de préférence sur toute la longueur. Le filetage 52 est adapté pour coopérer avec le taraudage 26. Le premier tronçon 25 du tube 18 reçoit la tige filetée 46. La tige filetée 46 est donc adaptée pour régler la longueur de la prothèse de hanche 12 par vissage de la tige filetée 46 dans le tube 18.

En variante, la partie articulée 16 comporte d'autres moyens de réglage et d'ancrage que le filetage 52 pour coopérer avec la partie d'ancrage 14 qui de son côté comportent des moyens complémentaires.

La tige filetée 46 présente un diamètre variable selon l'anatomie de la hanche. La tige filetée 46 comporte une première extrémité 54 à laquelle se raccorde la tige de connexion 48. La première extrémité 54 de la tige filetée 46 est orientée dans le même sens que la première extrémité 30 du tube 18. La tige filetée 46 comporte une seconde extrémité 56 qui porte une empreinte 58 adaptée pour coopérer avec un outil pour visser et dévisser la tige filetée 46 dans le tube 18. L'empreinte 58 est une empreinte positive, telle qu'une saillie, ou une empreinte négative, telle qu'une encoche. L'empreinte 58 est également adaptée pour recevoir un canon d'une prothèse du type « vis-plaque » permettant de traiter une fracture pertrochantérienne de survenue secondaire après l'arthroplastie.

La tige de connexion 48 comporte une partie cylindrique 60 et un cône morse 62.

La partie cylindrique 60 présente un diamètre sensiblement compris entre 12 et 16 mm, de préférence entre 13 et 15 mm. La partie cylindrique 60 est rectiligne (en traits faits sur la figure 3) ou coudée (non représenté). Dans le cas où la partie cylindrique 60 est coudée, la perforation centrale suit l'axe de rotation de chaque segment rectiligne de la partie cylindrique 60 coudée. La partie cylindrique 60 rentre en partie dans le tube 18.

La partie cylindrique 60 est insérée et coulisse dans le deuxième tronçon 27 suivant l'axe A-A et est en appui axial contre la tige filetée 46. La partie cylindrique 60 est indexée angulairement de telle sorte que la tige de connexion 48 se déplace linéairement le long de l'axe A-A sans tourner autour de l'axe A-A lorsque la tige filetée 46 est vissée ou dévissée dans le tube 18. L'indexation angulaire est obtenue par exemple en conférant à la partie de la partie cylindrique 60 qui rentre dans le tube 18 une section transversale asymétrique en rotation autour de l'axe A-A. Le deuxième tronçon 27 lisse présente une section transversale complémentaire et est adapté pour recevoir la partie cylindrique 60.

La partie de la partie cylindrique 60 qui rentre dans le deuxième tronçon 27 du tube 18 est par exemple de section transversale elliptique, rectangulaire ou rond avec une ou plusieurs empreintes d'indexation.

En variante, la partie cylindrique 60 est reliée à la tige filetée 46 par l'intermédiaire d'un système vis-écrou pour que la rotation de la tige filetée 46 entraîne la translation de la tige de connexion 46 le long de l'axe A-A.

Le choix de la forme (rectiligne ou coudée) de la partie cylindrique 60 dépend de la forme du fémur 2 pour lequel la prothèse de hanche 12 est utilisée. La partie cylindrique 60 angulaire est adaptée pour orienter la partie articulée 16 dans les différents plans, à savoir rétroversion, antéversion, valgus et varus. La partie cylindrique 60 linéaire est adaptée pour des cols 6 de fémur avec une anatomie standard antéversée.

Le cône morse 62 converge depuis la partie cylindrique 60 vers son extrémité libre. Le cône morse 62 est adapté pour recevoir la tête sphérique 17. Le cône morse 62 présente une forme standard. Ceci présente l'avantage que la partie articulée 16 peut être utilisée en combinaison avec des têtes sphériques standards existantes. La tige filetée 46 et la tige de connexion 48 sont métalliques. En variante, le cône morse 62 peut être remplacé par une autre pièce qui stabilise la tige de connexion 48 et la tête sphérique 17. Ladite autre pièce est par exemple un élément d'encliquetage.

En variante, la partie articulée 16 comporte la tige filetée 46 et le cône morse 62 espacé l'un de l'autre par la partie cylindrique 60 ou pas. Dans cette variante, la partie articulée 16 est rectiligne et d'un seul tenant.

Le centre de rotation de la tête sphérique 17 est ajustable par vissage et/ou dévissage de la tige filetée 46 dans le tube 18 de la partie d'ancrage 14.

La tête sphérique 17 comporte un logement 66 et un méplat 68. Le logement 66 présente une forme complémentaire à celle du cône morse 62 et est adapté pour recevoir le cône morse 62. Le méplat 68 s'étend dans un plan perpendiculaire à l'axe longitudinal du logement 66. Une fois que la tige de connexion 48 porte la tête sphérique 17, le méplat 68 est orienté en regard de la plaque d'appui 28.

La tête sphérique 17 est adaptée pour remplacer la tête de fémur 4. La tête sphérique 17 présente un diamètre variable qui dépend de la taille de l'articulation opérée et de l'opinion chirurgicale choisie par le chirurgien.

La tête sphérique 17 est de matière métallique ou céramique. La tête sphérique 17 est destinée à coopérer avec un cotyle (non représenté). Le cotyle est le cotyle naturel ou un cotyle prothétique.

La prothèse de hanche 12 comporte en outre un élément de blocage adapté pour bloquer la position de la partie articulée 16 par rapport à la partie d'ancrage 14 en bloquant la tige filetée 46 dans le tube 18. L'élément de blocage se présente par exemple sous forme du filetage 52 qui présente un angle d'hélice comme pour une vis irréversible. En variante (non représentée sur les figures), l'élément de blocage présente un élément individuel qui se situe à la seconde extrémité 56 de la tige filetée 46 ou au tube 18 taraudé au niveau de sa première extrémité 30.

La prothèse de hanche 12 est adaptée pour être stabilisée dans le col de fémur 6 grâce à la pièce de stabilisation primaire 34 (stabilisation primaire) et au revêtement ostéoinducteur qui recouvre toutes les surfaces de la prothèse de hanche 12 qui rentrent en contact avec l'os du col de fémur 6 (stabilisation secondaire).

La pose d'une prothèse de hanche 12 selon l'invention est décrite ci-dessous.

Dans une première étape, le patient est installé en décubitus dorsal. Une broche guide 72 de 1,5 à 2,5 mm de diamètre est introduite sous contrôle radioscopique dans le fémur 2 selon l'axe cervicale. La broche guide 72 passe d'une face externe 74 du fémur sous le massif trochantérien 8 à travers le massif trochantérien 8 centralement dans le col anatomique 6 jusqu'au centre de rotation de la tête de fémur 4. On mesure des distances qui serviront au réglage de la longueur de la prothèse de hanche 12 :
a) de la corticale du point de pénétration de la broche guide au centre de rotation de la tête de fémur 4
b) de la ligne intertrochantérienne 10 à la zone de coupe du col 6, ultérieurement présentant la surface de résection 32
c) de la zone de coupe du col 6 au centre de rotation de la tête de fémur 4.

Dans une deuxième étape, une tarrière cylindrique (non représentée) motorisée de diamètre sensiblement identique au diamètre externe du tube 18 est enfilée sur la broche guide 72, qui, guidée par la broche guide 72, fraise un canal 76 dans le fémur.

Dans une troisième étape, par voie antérieure, la tête de fémur 4 est sectionnée par une scie oscillante et on forme la surface de résection 32 sur laquelle prendra appui ultérieurement la plaque d'appui 28.

Dans une quatrième étape, la partie d'ancrage 14 est introduite par voie antérieure dans le canal 76 et ancrée dans le col de fémur 6. La plaque d'appui 28 prend appui sur la surface de résection 32.

Dans une cinquième étape, la partie cylindrique 60 de la tige de connexion 48 est au moins partiellement introduite dans la partie d'ancrage 14 par voie antérieure. La tige filetée 46 est vissée dans le tube 18 dans le sens opposé au moyen d'un outil adapté pour coopérer avec l'empreinte 58 afin d'approcher la tige filetée 46 à la tige de connexion 48.

Dans une sixième étape, la tête sphérique 17 est glissée dans l'articulation par voie antérieure et est positionné sur le cône morse 62 de la tige de connexion 48.

Dans une septième étape, par vissage et/ou dévissage de la tige filetée 46, la position de la partie articulée 16 par rapport à la partie d'ancrage 14 est ajustée. Cette position détermine la position du centre de rotation de la tête sphérique 17. On remesure les mêmes distances a), b) et c) que lors de la première étape. La broche guide 72 est laissée en place en région métaphysaire. Ensuite, la pièce de stabilisation primaire 34 est enfoncée, guidée par la broche guide 72 jusqu'à la plaque d'appui 28.

Dans une huitième étape, une fois que la longueur de la prothèse 12 est ajustée, l'élément de blocage vient bloquer la partie articulée 16 par rapport à la partie d'ancrage 14.

Ensuite, on procède aux étapes habituelles après la pose d'une prothèse de hanche.

La prothèse de hanche 12 selon l'invention présente une forme qui respecte les contraintes biomécaniques naturelles de l'os. La prothèse de hanche 12 permet de conserver le col de fémur anatomique 6 et donc la forme du fémur 2. La prothèse 12 permet d'éviter le « stress shielding » c'est-à-dire la réduction de la densité de l'os due à la présence de la prothèse qui réduit la sollicitation normale de l'os et de favoriser le renfort de la structure osseuse cervicale.

La prothèse de hanche 12 selon l'invention présente d'autres avantages. Les traumatismes musculaires lors de la pose de la prothèse de hanche 12 selon l'invention sont réduits. Par conséquent, le patient récupère rapidement après l'intervention chirurgicale et a besoin d'une rééducation courte. Globalement, l'utilisation de la prothèse de hanche 12 selon l'invention réduit les coûts liés à la pose et au traitement ultérieur.

En outre, grâce à son empreinte 58 prévu à la seconde extrémité 56 de la tige filetée 46 et grâce à la broche guide laissée en place, la prothèse de hanche 12 peut également servir, une fois posée dans le col de fémur 6, comme base pour la pose ultérieure de matériel complémentaire, par exemple du type vis plaque. Un tel scénario est envisageable après une fracture pertrochentérienne d'un fémur 2 dans lequel a été posée déjà une prothèse 12 selon l'invention.

Par ailleurs, grâce à la broche guide 72 laissée en place, il est possible d'accéder ultérieurement à la prothèse de hanche 12 pour des fins diagnostiques, telles qu'un prélèvement pour une étude bactériologique, ou pour des fins thérapeutiques, telles qu'un changement de la partie articulée 16.

## Revendications

1. Prothèse de hanche (12) comprenant :
- une partie d'ancrage (14) adaptée pour être ancrée dans un col de fémur (6) et comprenant un tube (18) comprenant un premier tronçon (25) taraudé adapté pour s'étendre dans le col de fémur (6) le long d'un axe (A-A),
- une tête sphérique (17) destinée à remplacer la tête de fémur (4), et
- une partie articulée (16) comprenant une tige filetée (46) de réglage s'étendant le long de l'axe (A-A) adaptée pour être vissée et/ou dévissée dans le tube (18), pour régler la position du centre de rotation de la tête sphérique (17) par rapport à la partie d'ancrage (14),
**caractérisée en ce que** la partie d'ancrage (14) est prévue pour s'étendre uniquement dans le col anatomique du fémur (6).

2. Prothèse (12) selon la revendication 1, dans laquelle la tige filetée (46) est prévue pour s'étendre uniquement dans le col anatomique (6).

3. Prothèse (12) selon la revendication 1 ou 2 dans laquelle la partie articulée (16) comprend une tige de connexion (48) adaptée pour recevoir la tête sphérique (17), et dans laquelle la tige filetée (46) et la tige de connexion (48) sont rectilignes et d'un seul tenant.

4. Prothèse (12) selon la revendication 1 ou 2, dans laquelle le tube (18) comprend un deuxième tronçon (27) lisse et dans laquelle la partie articulée (16) comprend une tige de connexion (48), la tige de connexion (48) étant adaptée pour être insérée et pour coulisser dans le deuxième tronçon (27) suivant l'axe (A-A) et étant en appui axial contre la tige filetée (46).

5. Prothèse (12) selon la revendication 4, dans laquelle la tige de connexion (48) est indexée angulairement de telle sorte que la tige de connexion (48) est adaptée pour se déplacer linéairement le long de l'axe (A-A) sans tourner autour de l'axe (A-A) lorsque la tige filetée (46) est vissée ou dévissée dans le tube (18).

6. Prothèse(12) selon la revendication 4 ou 5, dans laquelle la tige filetée (46) et la tige de connexion (48) s'étendent suivant un même axe (A-A).

7. Prothèse (12) selon la revendication 4 ou 5, dans laquelle la tige de connexion (48) présente une forme coudée.

8. Prothèse (12) selon l'une quelconque des revendications 1 à 7, dans laquelle la tige filetée (46) comporte à une seconde extrémité (56) une empreinte (58) adaptée pour coopérer avec un outil pour visser et dévisser la tige filetée (46) dans le tube (18) et pour coopérer avec un canon d'une prothèse complémentaire du type vis plaque, en cas d'une fracture pertrochantérienne après la pose de la prothèse (12).

9. Prothèse (12) selon l'une quelconque des revendications 1 à 8, comprenant un élément de blocage adapté pour bloquer la position de la tige filetée (46) par rapport au tube (18), notamment après réglage de la position du centre de rotation de la tête sphérique (17).

10. Prothèse (12) selon l'une quelconque des revendications 1 à 9, dans laquelle la partie d'ancrage (14) comprend une plaque d'appui (28) adaptée pour prendre appui sur une surface de résection (32) du col du fémur (6) résultant de la résection de la tête de fémur (4).

11. Prothèse (12) selon l'une quelconque des revendications 1 à 10 dans laquelle le tube (18) comporte sur sa surface extérieure (24) une pièce de stabilisation primaire (34 ) adaptée pour bloquer une rotation du tube (18) autour de l'axe (A-A) par rapport au col de fémur (6) et/ou un pivotement du tube (18) par rapport au col de fémur (6).

12. Prothèse (12) selon l'une quelconque des revendications 1 à 11, dans laquelle la partie d'ancrage (14) est au moins en partie recouverte d'un revêtement ostéoinducteur.
